Europäisches Patentamt

European Patent Office

Office européen des brevets

(11) Veröffentlichungsnummer: **0 472 088 A1**

# EUROPÄISCHE PATENTANMELDUNG

(21) Anmeldenummer: **91113534.1**

(51) Int. Cl.⁵: **A61M  39/00**, F16K 15/18

(22) Anmeldetag: **13.08.91**

(30) Priorität: **22.08.90 DE 4026524**

(43) Veröffentlichungstag der Anmeldung:
**26.02.92 Patentblatt  92/09**

(84) Benannte Vertragsstaaten:
**DE ES FR GB IT SE**

(71) Anmelder: **Wex, Roland**
**Am Hang 28**
**W-3508 Melsungen(DE)**

(72) Erfinder: **Wex, Roland**
**Am Hang 28**
**W-3508 Melsungen(DE)**

(74) Vertreter: **Ouermann, Helmut, Dipl.-Ing.**
**Postfach 6145 Gustav-Freytag-Strasse 25**
**W-6200 Wiesbaden(DE)**

(54) **Ventilanordnung für Flüssigkeitsleitungen an medizinischen Apparaten und Geräten oder dergleichen.**

(57) Die Erfindung betrifft eine Ventilanordnung für Flüssigkeitsleitungen an medizinischen Apparaten und Geräten oder dergleichen mit einem Ventilgehäuse (1) mit einer Einlaß- und einer Auslaßöffnung (20), einem im Ventilgehäuse angeordneten elastischen Ventilkörper (24), dessen Sitzfläche an einer ventilgehäuseseitigen Gegensitzfläche dichtend anliegt, wobei der Ventilkörper bei einem Einführen von Flüssigkeit durch die Einlaßöffnung von der Gegensitzfläche abgehoben ist.

Damit bei der Ventilanordnung nicht nur eine Durchströmung in einer Richtung, das heißt von der Einlaßzur Auslaßöffnung erfolgen kann, sondern auch die Möglichkeit besteht, über die Einlaßöffnung Flüssigkeit zu entnehmen, ist ein in die Einlaßöffnung einführbares Adapterstück (33) vorgesehen, das in eingeführter Position den Ventilkörper kontaktiert und mit dessen Sitzfläche (32) von der Gegensitzfläche des Gehäuses abhebt.

Fig. 1

Die Erfindung betrifft eine Ventilanordnung für Flüssigkeitsleitungen an medizinischen Apparaten und Geräten oder dergleichen, mit einem Ventilgehäuse mit einer Einlaß- und einer Auslaßöffnung, einem im Ventilgehäuse angeordneten elastischen Ventilkörper, dessen Sitzfläche an einer ventilgehäuseseitigen Gegensitzfläche dichtend anliegt, wobei der Ventilkörper bei einem Einführen von Flüssigkeit durch die Einlaßöffnung von der Gegensitzfläche abgehoben ist.

Derartige Ventilanordhungen sind in der Medizintechnik hinlänglich bekannt und dienen dem Zweck, eine Rückströmung von Flüssigkeit, das heißt von der Auslauföffnung zur Einlaßöffnung hin zu verhindern. So zeigt beispielsweise die EP 0 075 039 B1 ein die Rückströmung verhinderndes Rückschlagventil zur Verwendung an einer Infusionseinrichtung zum Zwecke der Überleitung medizinischer Flüssigkeiten. Dort ist der Ventilkörper als im Ventilgehäuse frei verschiebliche Scheibe ausgebildet, die ein geringeres spezifisches Gewicht als die Flüssigkeit aufweist, so daß sie die Neigung hat aufzuschwimmen und die oben befindliche Einlaßöffnung zu verschließen. Beim Überleiten der Flüssigkeit wird die Scheibe von der Einlaßöffnung weggedrückt. Aus der EP 0 224 641 A1 ist ein Injektionsventil bekannt, bei dem neben dem eigentlichen Flüssigkeitsdurchgang von einer Infusionseinrichtung zum Patienten ein quer hierzu angeordneter Anschlußstutzen vorgesehen ist, dessen Außenkonus mit einer Spritze verbindbar ist. Mittels dieser können zusätzlich Injektabilien verabreicht werden, wobei ein an der Innenwandung des Ventilgehäuses anliegender elastischer Ventilschlauch die Öffnung zum Anschlußstutzen verschließt und nur beim Zuführen der Flüssigkeit mittels der Spritze aufgrund des Spritzdruckes von der Wandung verdrängt wird.

Bei dem genannten Stand der Technik verhindern die Ventile zwar wirkungsvoll einen Rückfluß der Flüssigkeit in der Ventilanordnung, jedoch ist es bei diesen Ventilanordnungen nicht möglich, entgegen der Durchlaßrichtung des Ventiles Flüssigkeit zu entnehmen.

Es ist Aufgabe der vorliegenden Erfindung, eine Ventilanordnung der genannten Art so weiter zu bilden, daß bei dieser nicht nur eine Durchströmung in einer Richtung, das heißt von der Einlaßzur Auslaßöffnung erfolgen kann, sondern auch die Möglichkeit besteht, über die Einlaßöffnung Flüssigkeit zu entnehmen.

Die erfindungsgemäße Lösung ist gekennzeichnet durch ein in die Einlaßöffnung einführbares Adapterstück, das in eingeführter Position den Ventilkörper kontaktiert und dessen Sitzfläche von der Gegensitzfläche des Gehäuses abhebt.

Eine besondere Ausführungsform der Erfindung sieht vor, daß das Gehäuse einen rohrförmigen Ansatz mit einem Einlaßkanal aufweist, in den ein rohrförmiger Ansatz des Adapterstückes dichtend einsteckbar ist, wobei bei eingestecktem Adapterstück der rohrförmige Ansatz des Adapterstückes den Ventilkörper kontaktiert und abhebt, sowie im Ventilkörper oder im rohrförmigen Ansatz des Adapterstücks oder zwischen Ventilkörper und rohrförmigem Ansatz des Adapterstückes mindestens eine Überströmöffnung für die Flüssigkeit von der Auslaßöffnung zum rohrförmigen Ansatz des Adapterstückes gebildet ist. Die Erfindung ist nicht darauf beschränkt, daß das Adapterstück, das heißt der rohrförmige Ansatz des Adapterstückes von der Flüssigkeit durchströmt wird, es ist gleichfalls denkbar, den mit dem Ventilkörper in Kontakt gelangenden Bereich des Adapterstückes stiftförmig auszubilden, so daß dieser von der Flüssigkeit umströmt wird. Entscheidend ist nur, daß die Öffnungsbewegung, die beim Zuführen von Flüssigkeit die Position des Ventilkörpers verändert, beim Entnehmen von Flüssigkeit durch das Adapterstück aufrechterhalten wird. Das Adapterstück kann als eigenständiges Bauteil oder hinsichtlich seiner Funktion in eine Spritze integriert sein, im ersteren Fall müßte das Adapterstück zunächst mit einer Spritze verbunden werden und anschließend mit dem Ventilgehäuse, in letzterem Fall könnte die Spritze unmittelbar mit dem Ventilgehäuse verbunden werden.

Vorteilhaft ist der rohrförmige Ansatz des Adapterstückes in dem den Ventilkörper kontaktierenden Bereich mit mehreren radialen Überströmöffnungen versehen. Die Bildung der Überströmöffnungen im rohrförmigen Ansatz hat den Vorteil, daß diese bei der Herstellung des Adapterstückes einfach mitgestaltet werden können.

Die Innenkontur des rohrförmigen Ansatzes des Gehäuses ist zweckmäßig konisch ausgebildet und die Außenkontur des in das Gehäuse einführbaren Bereichs des rohrförmigen Ansatzes des Adapterstückes entsprechend konisch. Die genannte Formgebung ermöglicht ein einfaches, dichtes Zusammenführen von Ventilgehäuse und Adapterstück. Da nicht davon auszugehen ist, daß die Ventilanordnung grundsätzlich der Entnahme von Flüssigkeit dient, sollte der rohrförmige Ansatz des Gehäuses als weiblicher Luer-Lock-Ansatz ausgebildet sein, womit die Möglichkeit besteht, diesen mit einem entsprechenden männlichen Luer-Lock-Ansatz eine Infusionsleitung, einer Spritze oder dergleichen zu verbinden. Die dem Gehäuse abgewandte Bereich des rohrförmigen Ansatz des Adapterstückes sollte eine konische Innenkontur aufweisen, um in diesen eine Spritze zum Entnehmen von Flüssigkeit, wobei hier beispielsweise an das Entnehmen von Blut gedacht ist, einführen zu können. Ist der dem Gehäuse abgewandte Bereich des rohrförmigen Ansatzes des Adapterstückes zusätzlich als weiblicher Luer-Lock-Ansatz ausgebildet, besteht die Möglichkeit, eine einen männlichen Luer-Lock-Ansatz aufweisende Spritze auf das Adapterstück aufzuschrauben. Das Adapterstück sollte zusätzlich mit einem Griffbereich versehen sein, der zweckmäßig Bestandteil einer Hülse bildet, die im Mittelbereich des rohrförmigen Ansatzes des Adapterstückes über einen Steg mit dem rohrförmigen Ansatz verbunden ist. Die Hülse kann zusätzlich mit einem Schutzkappensitz für eine Schutzkappe versehen sein, die das in das Ventilgehäuse einsteckbare Ende des rohrförmigen Ansatzes des Adapterstückes abdeckt. Ist das andere Ende des rohrförmigen Ansatzes des Adapterstückes mit einem Luer-Lock-Ansatz versehen, kann auf diesen eine Schutzkappe aufgeschraubt werden.

Der Ventilkörper kann auf unterschiedliche Art und weise gestaltet sein und bei nicht einwirkendem Adapterstück sowohl ohne als auch mit Vorspannung die Einlaßöffnung des Ventilgehäuses verschließen. Eine besondere Ausführungsform der Erfindung sieht vor, daß der Ventilkörper aus elastischem Material besteht und bei nicht einwirkendem Adapterstück unter Vorspannung die Einlaßöffnung verschließt. Insbesondere sollte bei nicht einwirkendem Adapterstück der Ventilkörper im Ventildichtbereich schirmförmig ausgebildet sein und der Schirmrand des Hohlkörpers dessen Sitzfläche darstellen, sowie bei einwirkendem Adapterstück dieses den Ventilschirm im Bereich des Ventilschirminnenkegels kontaktieren. Durch diese Ausbildung des Ventilkörpers ergibt sich eine extrem geringe Dichtfläche, hierdurch gute Flächenpressung bei rückwärtigem Druckaufbau, sowie geringe Adhäsionskräfte, was insbesondere bei zucker- oder fetthaltigen Lösungen von Bedeutung ist. Als elastisches Material für den Ventilkörper kommt vorzugsweise PUR oder Silicon in Frage.

Die geometrische Anordnung von Adapterstück und Ventilgehäuse sollte so bemessen sein, daß der Ventilschirm bei einwirkendem Adapterstück eine im wesentlichen ebene Form einnimmt und in dieser Stellung der Durchmesser des Ventilschirms geringer ist als die Innendurchmesser des Gehäuses im zugeordneten Gehäusebereich. Somit kann beim Entnehmen der Flüssigkeit diese zwischen dem Ventilschirm und der Gehäuseinnenwandung strömen und durch das Adapterstück gelangen, insbesondere durch zwei diametral angeordnete Überströmöffnungen im rohrförmigen Ansatz des Adapterstückes.

Der Ventilkörper ist zweckmäßig durch einen im Gehäuse gehaltenen Ventilkegel und den Ventilschirm gebildet, wobei sich der Ventilkegel zum Ventilschirm hin verjüngt. Die Aufstandsfläche des Ventilkegels sorgt damit für eine relativ großflächige Lagerung des Ventilkörpers auf der der Einlaßöffnung gegenüberliegenden Seite des Ventilgehäuses. Die Aufstandsfläche des Ventilkegels ist vorteilhaft kreisförmig, deckt die Auslaßöffnung des Gehäuses ab und ist mit mindestens einer Durchflußnut versehen, die den Gehäusehohlraum, der den Kegelmantel umgibt, mit der Auslaßöffnung verbindet. Beim Entnehmen von Flüssigkeit gelangt diese somit von der Auslaßöffnung durch den Bodenbereich des Ventilkegels, nämlich durch insbesondere zwei diametral angeordnete Durchflußnuten und umströmt von dort die Kegelwandung des Ventilkegels sowie den in der ebenen Stellung befindlichen Ventilschirm zur Einlaßöffnung.

Zur optimalen Lagerung des Ventilkörpers im Gehäuse sollte der Ventilkegel im Bereich der Aufstandsfläche mit einem zylindrischen Ansatz versehen sein und dessen Außendurchmesser dem Innendurchmesser des Gehäuses in diesem Gehäusebereich entsprechen, so daß der Ventilkörper leicht aber dennoch mit nur geringem Spiel in das Gehäuse einsetzbar ist und somit eine definierte Position des Schirmrandes des Ventilschirmes zum Ventilgehäuse und damit eine optimale Dichtigkeit sichergestellt ist. Die Durchflußnuten des Ventilkegels sollten in diesem Fall vom konischen Bereich des Ventilkegels zur Auslaßöffnung des Gehäuses verlaufen.

Als bevorzugtes Anwendungsgebiet der erfindungsgemäßen Ventilanordnung wird es gesehen, wenn diese Bestandteil eines Infusionssystems bildet, mit einem ersten Gehäuseteil mit einer Zuflußöffnung zum Verbinden mit einer Infusionszuleitung und einer Abflußöffnung zum Verbinden mit einer patientenseitigen Leitung, wobei das erste Gehäuseteil mindestens eine die Auslaßöffnung der Ventilanordnung darstellende Überströmöffnung aufweist, an das erste Gehäuseteil im Bereich der Überströmöffnung sich ein den Ventilkörper aufnehmende zweite Gehäuseteil anschließt und mit der Einlaßöffnung dieses Gehäuseteils das Adapterstück sowie mit diesem eine Spritze für die Entnahme von Flüssigkeit, insbesondere zur Entnahme von Blut, verbindbar ist. Die erfindungsgemäße Ventilanordnung wird somit von der eigentlichen Infusionsflüssigkeit nicht durchströmt, sondern dient ausschließlich dem Zuführen bzw. Entnehmen anderer Flüssigkeiten, beispielsweise für zusätzliche Injektionen oder der Entnahme von Blut. Eine vorteilhafte Ausgestaltung der Erfindung sieht vor, daß eine weitere Überströmöffnung in eine weitere Ventilanordnung mündet, sowie mit dieser Ventilanordnung ein Filter zum gefilterten Zuspritzen von Medikamenten verbindbar ist.

Weitere Merkmale der Erfindung sind in der Beschreibung der Figuren und in den Unteransprüchen dargestellt, wobei bemerkt wird, daß alle Einzelmerkmale und alle Kombinationen von Einzelmerkmalen weitere erfinderische Ausgestaltungen darstellen.

In den Figuren ist die Erfindung an zwei Ausführungsformen beispielsweise dargestellt, ohne auf diese beschränkt zu sein. Es stellt dar:

Figur 1 die erfindungsgemäße Ventilanordnung in einer ersten Ausführungsform, gezeigt in einem Mittellängsschnitt bei in das Ventilgehäuse eingesetztem Adapterstück,

Figur 2 das Ventilgehäuse der in Figur 1 gezeigten Ventilanordnung in einem Mittellängsschnitt (bei entferntem Adapterstück),

Figur 3 einen Schnitt durch das Ventilgehäuse gemäß der Linie A-A in Figur 2,

Figur 4 einen Längsmittelschnitt durch das Adapterstück und

Figur 5 die Ventilanordnung in einer zweiten Ausführungsform, bei der das Ventilgehäuse modifiziert ist und Bestandteil eines mehrere Anschlüsse aufweisenden Infusionssystems bildet.

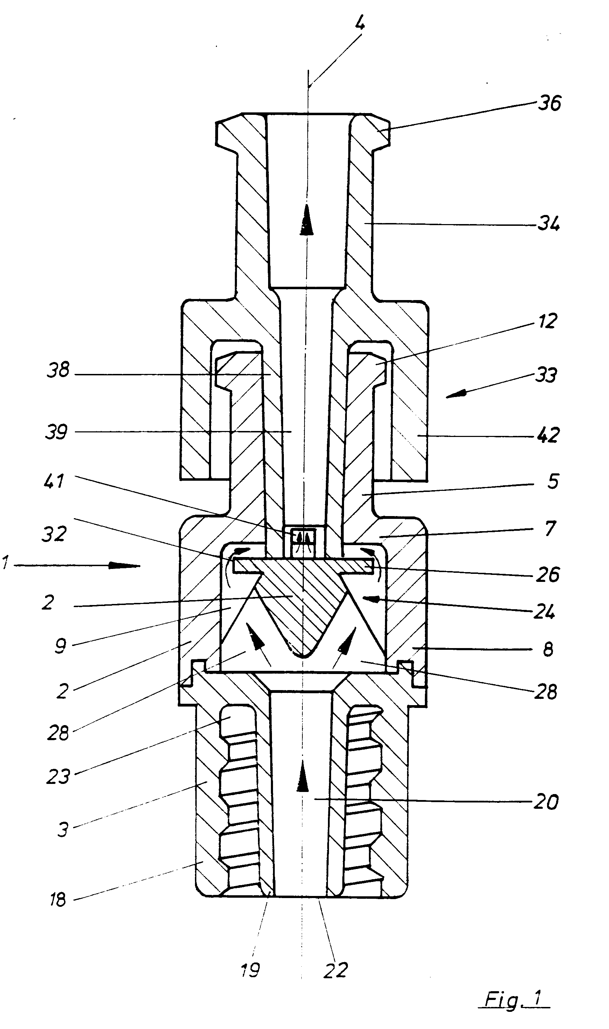

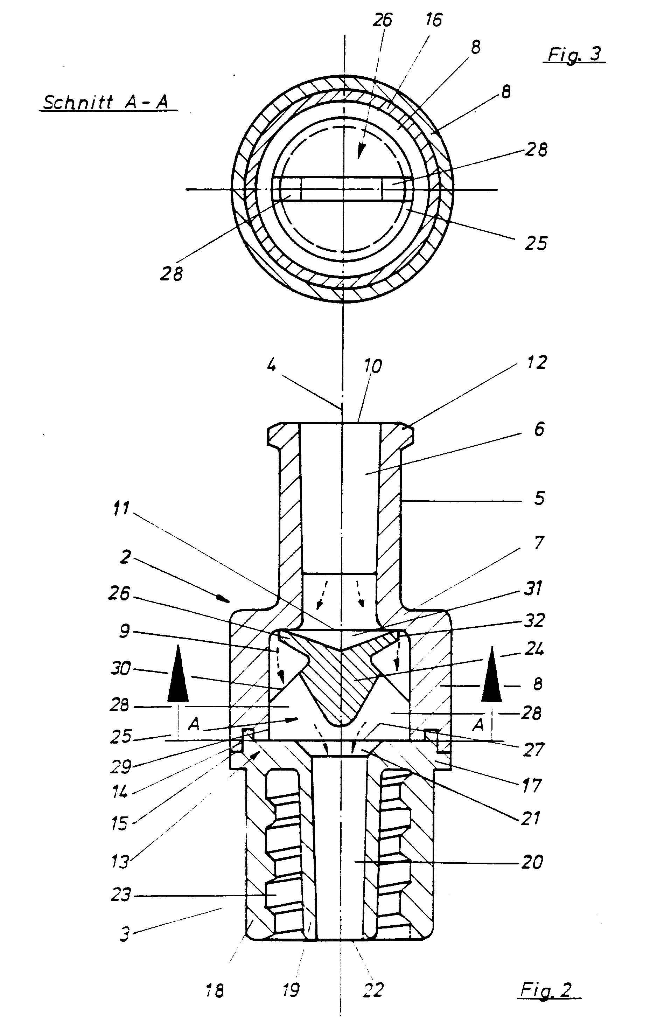

Die Figuren 1 bis 4 zeigen einen Ventilgehäuse 1, das aus zwei Teilen 2 und 3 besteht. Wie die Figuren 1 und 2 verdeutlichen, ist das Ventilgehäuseteil 2 rotationssymmetrisch zur Achse 4 ausgebildet, es weist einen rohrförmigen Ansatz 5, der einen Einlaßkanal 6 bildet, auf, über einen sich radial erstreckenden Abschnitt 7 ist der rohrförmige Ansatz 5 mit einem erweiterten ringförmigen Ansatz 8 verbunden, der einen Hohlraum 9 einschließt. Die Innenkontur des rohrförmigen Ansatzes 5 ist konisch ausgebildet, sie verjüngt sich von der äußeren Öffnung 10 zur in den ringförmigen Ansatz 8 führenden Mündungsöffnung 11, der Hohlraum 9 weist die Form eines Zylinders auf. Das Ventilgehäuseteil 2 ist im Bereich der äußeren Öffnung 10 mit einem weiblichen Luer-Lock-Ansatz 12 versehen, das diesem abgewandte Ende weist eine Hinterschneidung 13 mit einer Ringnut 14 auf. Diese dienen der Aufnahme des anderen Ventilgehäuseteiles 3, bei dem ein äußerer hinterschnittener Bereich 15 mit dem nicht hinterschnittenen Bereich des Ventilgehäuseteiles 2 korrespondiert sowie ein Ringansatz 16 mit der Ringnut 14. Das Ventilgehäuse 3 ist durch eine zentrale Ringscheibe 17, die auf ihrer dem Ventilgehäuseteil 2 zugewandten Seite den Ringansatz 16 aufweist, sowie einen äußeren rohrförmigen Ansatz 18 und einen inneren rohrförmigen Ansatz 19 mit Auslaßkanal 20 gebildet. Der innere rohrförmige Ansatz 19 ist konisch ausgebildet, er verjüngt sich von seiner dem Hohlraum 9 zugewandten inneren Öffnung 21 zur äußeren Öffnung 22. Der äußere rohrförmige Ansatz 18 ist mit einem Innengewinde 23 versehen, so daß das Ventilgehäuseteil 3 sich als männlicher Luer-Lock-Ansatz darstellt.

Beide Ventilgehäuseteile 2 und 3 bestehen aus durchsichtigem Kunststoff, sie werden nach dem Einlegen eines nach Aufbau und Funktionsweise noch näher zu beschreibenden Ventilkörpers 24 ineinander gesteckt, wobei der Ringansatz 16 die Ringnut 14 durchsetzt, und dann unlösbar miteinander verschweißt.

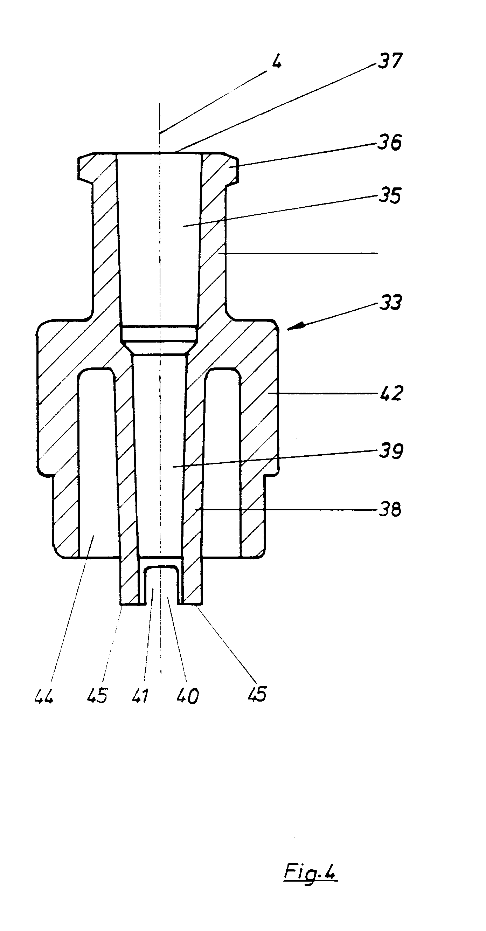

Der Ventilkörper besteht aus einem elastischem Material, beispielsweise PUR oder Silicon. Wie der Darstellung der Figur 2 zu entnehmen ist (bei nicht einwirkendem Adapterstück), ist der einteilige Ventilkörper 24 durch einen Ventilkegel 25 und einen Ventilschirm 26 gebildet, wobei sich der Ventilkegel 25 zum Ventilschirm 26 hin verjüngt. Der Ventilkegel 25 deckt mit seiner kreisförmigen Aufstandsfläche 27 die innere Öffnung 21 zwischen den beiden Ventilgehäuseteilen 2 und 3 weitgehend ab, das heißt er ist mit zwei Durchflußnuten 28 (siehe auch Figur 3) versehen, die den Bereich zwischen dem Ventilkegel 25 und dem Ventilschirm 26 mit der Öffnung 21 verbinden. Der Ventilkegel 25 ist dabei im Bereich seiner Aufstandsfläche 27 mit einem zylindrischen Ansatz 29 versehen, dessen Außendurchmesser dem Durchmesser des Hohlraumes 9 entspricht. Die Durchflußnuten 28 verlaufen jeweils vom eigentlichen konischen Bereich 30 des Ventilkegels 25 zur Öffnung 21. Der Ventilschirm 26 schließt mit dem konischen Bereich 30 des Ventilkegels 25 einen spitzen Winkel ein, sein Schirm-Innenkegel 31 ist stumpfwinklig ausgebildet, wobei sich die Stärke des Schirmes vom Rand nach innen hin zwecks Stabilisierung der Rückstellkraft des Schirmes vergrößert. Die Längsabmessung des Ventilkörpers 24, das heißt seine Abmessung in Richtung der Achse 4 ist so gewählt, daß der Ventilschirm 26 bei Anlage des Ventilkegels 25 am Ventilgehäuseteil 3 mit seinem Schirmrand 32 unter Vorspannung am Abschnitt 7 des Ventilgehäuseteiles 2 anliegt und so den Einlaßkanal 6 gegenüber dem Hohlraum 9 abdichtet. Der Bereich des Hohlraumes 9 zwischen dem Ventilschirm 26 und dem Ventilkegel 25 stellt den Bewegungsraum für den Ventilschirm 26 dar, dessen Außendurchmesser kleiner ist als der Außendurchmesser des Ansatzes 29 des Ventilkegels 25.

In einem konkreten Anwendungsfall wird die in Figur 2 gezeigte Ventilanordnung im Bereich ihres rohrförmigen Ansatzes 5 mit einer nicht gezeigten Spritze verbunden, das heißt deren Spritzenkegel in den innen konisch zulaufenden rohrförmigen Ansatz 5 eingesteckt. Mit dem männlichen Luer-Lock-Ansatz des Ventilgehäuseteiles 3 wird ein nicht gezeigter weiblicher Luer-Lock-Ansatz verbunden, der in Fließverbindung mit einer zur Vene eines Patienten führenden, gleichfalls nicht gezeigten Kanüle steht. Zum Zuführen von Injektionsflüssigkeit wird die Spritze betätigt, wobei der aufgebaute Spritzendruck den Ventilschirm 26 in Richtung des Ventilkegels 25 vom Abschnitt 7 wegbewegt, so daß die Injektionsflüssigkeit gemäß der gestrichelten Pfeile in Figur 2 außen am Ventilschirm 26 vorbei und durch die beiden Durchflußnuten in den Auslaßkanal 20 und damit zum Patienten gelangen kann. Sobald die Injektionsspritze nicht mehr betätigt wird, sinkt der Flüssigkeitsdruck innerhalb des Ventilgehäuses 1, so daß sich der Ventilschirm 26 aufgrund des Rückstellverhaltens des Ventilkörpers 24 wieder gegen den Abschnitt 7 des Ventilgehäuseteiles 2 anlegt. Ungeachtet dessen würde selbstverständlich auch ein Ansteigen des Flüssigkeitsdrucks im Auslaßkanal 20 über das Niveau des Einlaßkanales 6 zu einem Schließen des Ventiles in Art eines Rückschlagventiles führen.

Figur 1 verdeutlicht den Einsatz eines gleichfalls aus Kunststoff bestehenden Adapterstückes 33 bei der insoweit beschriebenen Ventilanordnung, das es ermöglicht, den Ventilschirm 26 durch körperliche Einwirkung in der vom Abschnitt 7 abgehobenen, ebenen Position dauerhaft zu halten, so daß Flüssigkeit, beispielsweise Blut, vom Patienten in eine Injektionsspritze eingesogen werden kann. Es wird dabei davon ausgegangen, daß nach wie vor der Auslaßkanal 20 mit einer Venenkanüle verbunden ist und in den Einlaßkanal 6 eine Spritze eingesteckt ist.

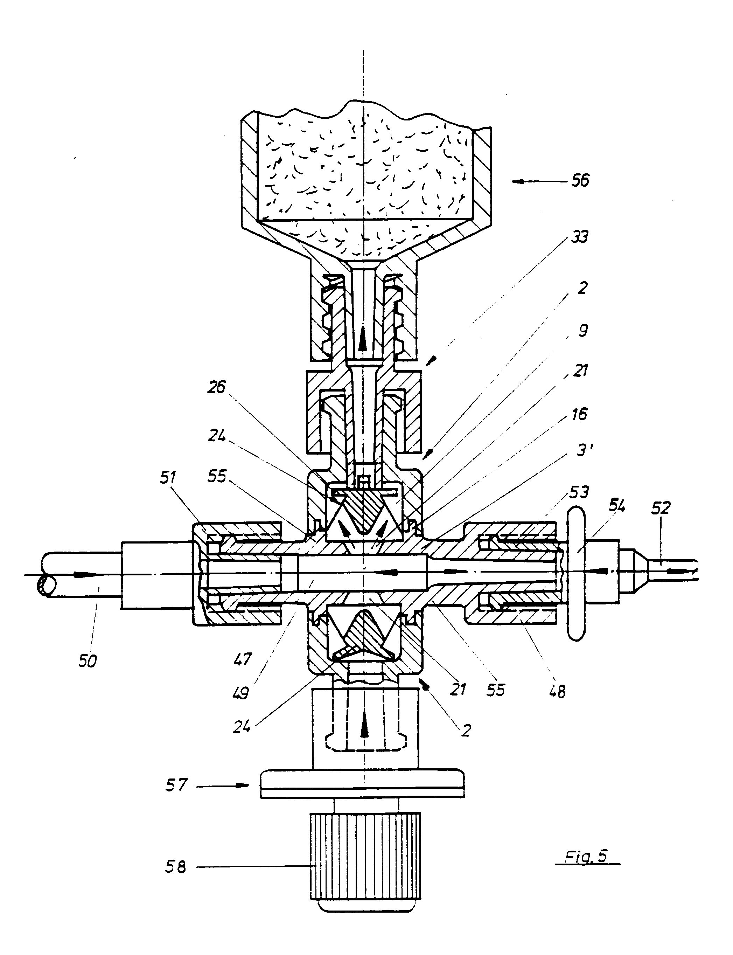

Der Figur 4 ist zu entnehmen, daß das Adapterstück 33 entsprechend der Ausbildung des Ventilgehäuses 1 im Bereich des rohrförmigen Ansatzes 5 einen rohrförmigen Ansatz 34 mit Einlaßkanal 35, weiblichen Luer-Lock-Ansatz 36 und äußerer Öffnung 37 aufweist. An den rohrförmigen Ansatz 34 schließt sich ein rohrförmiger Ansatz 38 an, durch den ein in Fließverbindung mit dem Einlaßkanal 35 stehender Kanal 39 geführt ist. Im Bereich dessen äußerer Öffnung 40 ist der rohrförmige Ansatz 38 mit einer Radialkerbe 41 versehen, so daß zwei gegenüberliegende Ausnehmungen im Öffnungsbereich des rohrförmigen Ansatzes 38 gebildet sind. Einlaßkanal 35 und Kanal 39 sind jeweils konisch ausgebildet, der Einlaßkanal 35 verjüngt sich zum Kanal 39 hin und der Kanal 39 vom Einlaßkanal 35 zur äußeren Öffnung 40. Die Innenkontur des Einlaßkanales 35 entspricht der Innenkontur des Einlaßkanals 6 und die Außenkontur des rohrförmigen Ansatzes 38 wiederum der Innenkontur des Einlaßkanales 6. Im Übergang von rohrförmigem Ansatz 34 zum rohrförmigem Ansatz 38 ist eine sich konzentrisch zum rohrförmigen Ansatz 38 erstreckende Griffhülse 42 über einen Abschnitt 43 mit dem Verbindungsbereich verbunden. Der rohrförmige Ansatz 35 und die Griffhülse 42 schließen zwischen sich eine Ausnehmung 44 ein und fungieren als männlicher Luer-Ansatz.

Zum Überführen der Ventilanordnung in die in Figur 1 gezeigte Entnahmestelle wird das Adapterstück mit seinem rohrförmigen Ansatz 38 in den rohrförmigen Ansatz 5 des Ventilgehäuses 1 eingesteckt, so daß die Außenfläche des außen konischen Ansatzes in den konischen Einlaßkanal 6 des Ventilgehäuses 1 kontaktiert und entsprechend der weibliche Luer-Lock-Ansatz 12 das Adapterstück 33. Kurz vor der Kontaktierung von Adapterstück 33 und Ventilgehäuse 1 gelangt die vordere Ringfläche 45 des rohrförmigen Ansatzes 38 in Anlage mit dem Ventilschirm 26, so daß dieser beim vollständigen Einschieben des Adapterstückes 33 in das Ventilgehäuse 1 in die in Figur 1 gezeigte ebene Gestalt überführt wird und wegen der Verbindung von Adapterstück 33 und Ventilgehäuse 1 in dieser Position verbleibt. Entgegen der Wirkrichtung des Rückschlagventiles kann dann Blut bzw. Flüssigkeit entnommen werden, die gemäß der Pfeile in Figur 1 durch den Auslaßkanal 20, die Durchflußnuten 28 am Ventilschirm 26 vorbeiströmt, von dort durch die der Radialkerbe 41 zugeordneten beiden Öffnungen zwischen dem rohrförmigen Ansatz 38 und den Ventilschirmen 26 in den Kanal 39 einströmt und von dort über den Einlaßkanal 35 in die Spritze gelangt. Nach dem Entfernen des Adapterstückes führt das Rückstellverhalten des Ventilkörpers 24 wieder dazu, daß der Ventilschirm 26 den Durchfluß im Bereich des Abschnittes 7 verschließt.

Bei der in Figur 5 gezeigten Ausführungsform bildet die erfindungsgemäße Ventilanordnung Bestandteil eines Infusionssystems und ist in Art eines Doppelventiles ausgeführt. Die Figur zeigt ein Ventilgehäuseteil 3' mit einem weiblichen Luer-Lock-Ansatz 47 und einem männlichen Luer-Lock-Ansatz 48. Im Bereich des Ansatzes 47 ist eine Zuflußöffnung, im Bereich des Ansatzes 48 eine Abflußöffnung gebildet, die durch einen Kanal 49 miteinander verbunden sind. Mit dem weiblichen Luer-Lock-Ansatz 47 ist eine Infusionszuleitung 50 über einen männlichen Luer-Lock-Ansatz 51 verbunden, mit dem männlichen Luer-Lock-Ansatz 48 ein Kanülenrohr 52 über einen weiblichen Luer-Lock-Ansatz 53. Mit der Bezugsziffer 54 ist eine Griffplatte der Venenverweilkanüle bezeichnet. Das Gehäuseteil 3' ist im Mittelbereich des Kanales 49 mit zwei gegenüberliegend angeordneten inneren Öffnungen 21 versehen, die damit radial zum Durchgang durch das Gehäuseteil 3' von der Infusionszuleitung 50 zum Kanülenrohr 52 positioniert sind. Die jeweilige Öffnung 21 umgibt ein ringförmiger Lagerflansch 55, der, wie die Ringscheibe 17 der Ausführungsform nach den Figuren 1 bis 4 gestaltet ist, somit eine Hinterschneidung 15 und einen Ringansatz 16 aufweist. Mit jedem Lagerflansch 55 ist ein Ventilgehäuseteil 2 gemäß der zuvor beschriebenen Ausführungsform verschweißt, der zwischen dem Lagerflansch 55 und dem Ventilgehäuseteil 2 gebildete Hohlraum 9 nimmt wiederum den Ventilkörper 24 auf. Wie der Darstellung der Figur 5 zu entnehmen ist, ist auf das obere Ventilgehäuseteil 2 ein Adapterstück 33 aufgesteckt und auf dieses wiederum eine Spritze 56 zur Blutentnahme. Infolge der Einwirkung des Adapterstückes 33 ist der Ventilschirm 26 in seine gestreckte, ebene Position überführt, bei der Blut aus dem Kanal 49 in die Spritze 56 aufgezogen werden kann. Auf den weiblichen Luer-Lock-Ansatz des unteren Gehäuseteiles 2 ist ein Zuspritzfilter 57 aufgesteckt, der dem gefilerten Zuspritzen von Medikamenten dient. Bei nicht benutztem Zuspritzfilter 57 ist dieser mit einem Verschlußkonus 58 verschlossen, der dort gezeigte Ventilkörper 24 befindet sich in der Schließstellung.

**Bezugszeichenliste**

| | |
|---|---|
| 1 | Ventilgehäuse |
| 2 | Ventilgehäuseteil |
| 3 | Ventilgehäuseteil |
| 3' | Ventilgehäuseteil |
| 4 | Achse |
| 5 | rohrförmiger Ansatz |
| 6 | Einlaßkanal |
| 7 | Abschnitt |
| 8 | ringförmiger Ansatz |
| 9 | Hohlraum |
| 10 | äußere Öffnung |
| 11 | Mündungsöffnung |
| 12 | weiblicher Luer-Lock-Ansatz |
| 13 | Hinterschneidung |
| 14 | Ringnut |
| 15 | Hinterschneidung |
| 16 | Ringansatz |
| 17 | Ringscheibe |
| 18 | äußerer rohrförmiger Ansatz |
| 19 | innerer rohrförmiger Ansatz |
| 20 | Auslaßkanal |
| 21 | innere Öffnung |
| 22 | äußere Öffnung |
| 23 | Innengewinde |
| 24 | Ventilkörper |
| 25 | Ventilkegel |
| 26 | Ventilschirm |
| 27 | Aufstandsfläche |
| 28 | Durchflußnut |
| 29 | Ansatz |
| 30 | konischer Bereich |
| 31 | Schirminnenkegel |
| 32 | Schirmrand |
| 33 | Adapterstück |
| 34 | rohrförmiger Ansatz |
| 35 | Einlaßkanal |
| 36 | weiblicher Luer-Lock-Ansatz |
| 37 | äußere Öffnung |
| 3 8 | rohrförmiger Ansatz |
| 39 | Kanal |
| 40 | äußere Öffnung |
| 41 | Radialkerbe |
| 42 | Griffhülse |
| 43 | Abschnitt |
| 44 | Ausnehmung |
| 45 | vordere Ringfläche |
| 46 | Gehäuse |
| 47 | weiblicher Luer-Lock-Ansatz |
| 48 | männlicher Luer-Lock-Ansatz |
| 49 | Kanal |
| 50 | Infusionszuleitung |
| 51 | männlicher Luer-Lock-Ansatz |
| 52 | Kanülenrohr |
| 53 | weiblicher Luer-Lock-Ansatz |
| 54 | Griffplatte |
| 55 | Lagerflansch |
| 56 | Spritze |
| 57 | Zuspritzfilter |
| 58 | Verschlußkonus |

**Patentansprüche**

1. Ventilanordnung für Flüssigkeitsleitungen an medizinischen Apparaten und Geräten oder dergleichen, mit einem Ventilgehäuse mit einer Einlaß- und einer Auslaßöffnung, einem im Ventilgehäuse angeordneten elastischen Ventilkörper, dessen Sitzfläche an einer ventilgehäuseseitigen Gegensitzfläche dichtend anliegt, wobei der Ventilkörper bei einem Einführen von Flüssigkeit durch die Einlaßöffnung von der Gegensitzfläche abgehoben ist, **gekennzeichnet** durch ein in die Einlaßöffnung (6) einführbares Adapterstück (33), das in eingeführter Position den Ventilkörper (24) kontaktiert und mit dessen Sitzfläche (32) von der Gegensitzfläche (7) des Gehäuses (1) abhebt.

2. Ventilanordnung nach Anspruch 1, **dadurch gekennzeichnet**, daß das Gehäuse (1) einen rohrförmigen Ansatz (5) mit einem Einlaßkanal (6) aufweist, in den ein rohrförmiger Ansatz (38) des Adapterstückes (33) dichtend einsteckbar ist, wobei bei eingestecktem Adapterstück (33) der rohrförmige Ansatz (38) des Adapterstückes (33) den Ventilkörper (24) kontaktiert und abhebt, sowie im Ventilkörper (24) oder im rohrförmigen Ansatz (38) des Adapterstückes (33) oder zwischen Ventilkörper (24) und rohrförmigem Ansatz (38) des Adapterstückes (33) mindestens eine Überströmöffnung (41) für die Flüssigkeit von der Auslaßöffnung (21) zum rohrförmigen Ansatz (38) des Adapterstückes (33) gebildet ist.

3. Ventilanordnung nach Anspruch 2, **dadurch gekennzeichnet**, daß der rohrförmige Ansatz (38) des Adapterstückes (33) in dem den Ventilkörper (24) kontaktierenden Bereich mit radialen Überströmöffnungen (41) versehen ist.

4.  Ventilanordnung nach Anspruch 2 oder 3, **dadurch gekennzeichnet**, daß die Innenkontur des rohrförmigen Ansatzes (5) des Gehäuses (1) konisch ausgebildet ist und die Außenkontur des in das Gehäuse (1) einführbaren Bereichs des rohrförmigen Ansatzes (38) des Adapterstückes (33) entsprechend konisch.

5.  Ventilanordnung nach Anspruch 4, **dadurch gekennzeichnet**, daß der rohrförmige Ansatz (5) des Gehäuses (1) als weiblicher Luer-Lock-Ansatz (12) ausgebildet ist.

6.  Ventilanordnung nach Anspruch 4 oder 5, **dadurch gekennzeichnet**, daß der dem Gehäuse (1) abgewandte Bereich des rohrförmigen Ansatzes (34) des Adapterstückes (33) eine konische Innenkontur aufweist.

7.  Ventilanordnung nach Anspruch 6, **dadurch gekennzeichnet**, daß der dem Gehäuse (1) abgewandte Bereich des rohrförmigen Ansatzes (34) des Adapterstückes (33) als weiblicher Luer-Lock-Ansatz (36) ausgebildet ist.

8.  Ventilanordnung nach einem der Ansprüche 2 bis 7, **dadurch gekennzeichnet**, daß das Adapterstück (33) mit einem Griffbereich (42) versehen ist.

9.  Ventilanordnung nach einem der Ansprüche 1 bis 8, **dadurch gekennzeichnet**, daß der Ventilkörper (24) aus elastischem Material besteht und bei nicht einwirkendem Adapterstück (33) unter Vorspannung die Einlaßöffnung (6) verschließt.

10. Ventilanordnung nach einem der Ansprüche 1 bis 9, **dadurch gekennzeichnet**, daß bei nicht einwirkendem Adapterstück (33) der Ventilkörper (24) im Ventildichtbereich schirmförmig ausgebildet ist und der Schirmrand (32) des Ventilkörpers (24) dessen Sitzfläche darstellt, sowie bei einwirkendem Adapterstück (33) dieses den Ventilschirm (26) im Bereich des Ventilschirminnenkegels (31) kontaktiert.

11. Ventilanordnung nach Anspruch 10, **dadurch gekennzeichnet**, daß der Ventilschirm (26) bei einwirkendem Adapterstück (33) eine im wesentlichen ebene Form einnimmt und der Durchmesser des ebenen Ventilschirms (26) geringer ist als der Innendurchmesser des Gehäuses (1) im zugeordneten Gehäusebereich (8).

12. Ventilanordnung nach Anspruch 10 oder 11, **dadurch gekennzeichnet**, daß der Ventilkörper (24) durch einen im Gehäuse (1) gehaltenen Ventilkegel (25) und den Ventilschirm (26) gebildet ist, wobei sich der Ventilkegel (25) zum Ventilschirm (26) hin verjüngt.

13. Ventilanordnung nach Anspruch 12, **dadurch gekennzeichnet**, daß der Ventilkegel (25) mit seiner kreisförmigen Aufstandsfläche (27) die Auslaßöffnung (21) des Gehäuses (1) abdeckt und mit mindestens einem Durchfluß (28) versehen ist, der den Gehäusehohlraum (9), der den Kegelmantel umgibt, mit der Auslaßöffnung (21) verbindet.

14. Ventilanordnung nach Anspruch 13, **dadurch gekennzeichnet**, daß der Ventilkegel (25) im Bereich der Aufstandsfläche (27) mit einem zylindrischen Ansatz (29) versehen ist und dessen Außendurchmesser dem Innendurchmesser des Gehäuses (1) in diesem Gehäusebereich (8) entspricht.

15. Ventilanordnung nach Anspruch 14, **dadurch gekennzeichnet**, daß mehrere, sich jeweils vom konischen Bereich (30) des Ventilkegels (25) zur Auslaßöffnung (21) des Gehäuses (1) verlaufende Durchflußnuten (28) im Ventilkegel (25) vorgesehen sind.

16. Ventilanordnung nach einem der Ansprüche 1 bis 15, **dadurch gekennzeichnet**, daß sie Bestandteil eines Infusionssystems bildet, mit einem ersten Gehäuseteil (3') mit einer Zuflußöffnung zum Verbinden mit einer Infusionsleitung (50) und einer Abflußöffnung zum Verbinden mit einer patientenseitigen Leitung (52), wobei das erste Gehäuseteil (3') mindestens eine die Auslaßöffnung der Ventilanordnung darstellende Überströmöffnung (21) aufweist, an das erste Gehäuseteil (3') im Bereich der Überströmöffnung (21) sich das den Ventilkörper (24) aufnehmende zweite Gehäuseteil (2) anschließt und mit der Einlaßöffnung (6) dieses Gehäuseteiles (2) das Adapterstück (33) sowie mit diesem eine Spritze (56) für die Entnahme von Flüssigkeit, insbesondere zur Entnahme von Blut, verbindbar ist.

17. Ventilanordnung nach Anspruch 16, **dadurch gekennzeichnet**, daß eine weitere Überströmöffnung (21) in eine weitere Ventilanordnung mündet, sowie mit dieser Ventilanordnung ein Filter (57) zum gefilterten Zuspritzen von Medikamenten verbindbar ist.

*Fig. 1*

Fig. 3

Schnitt A - A

Fig. 2

*Fig.4*

Fig. 5

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| X,Y | EP-A-0 261 317 (B BRAUN MELSUNGEN AG)<br>* Spalte 6, Zeile 24 - Zeile 57; Abbildungen *<br>– – – | 1-15,16,<br>17 | A 61 M 39/00<br>F 16 K 15/18 |
| Y | FR-A-2 316 970 (THE KENDALL COMPANY)<br>* Seite 5, Zeile 33 - Seite 6, Zeile 30; Abbildungen *<br>– – – | 16,17 | |
| A | GB-A-2 067 075 (FRESENIUS)<br>* Zusammenfassung; Abbildungen *<br>– – – | 1,4-9 | |
| A | DE-A-3 618 739 (CITTERIO)<br>* Zusammenfassung; Abbildungen 1,6 *<br>– – – | 1,4-9 | |
| A | FR-A-1 552 417 (LATEX PRODUCTS)<br>* Ansprüche 1-6; Abbildungen *<br>– – – – – | 1-7 | |

|  |
|---|
| RECHERCHIERTE SACHGEBIETE (Int. Cl.5) |
| A 61 M<br>F 16 K<br>F 16 L |

**Der vorliegende Recherchenbericht wurde für alle Patentansprüche erstellt**

| Recherchenort | Abschlußdatum der Recherche | Prüfer |
|---|---|---|
| Den Haag | 17 Oktober 91 | CLARKSON P.M. |